# EUROPEAN PATENT SPECIFICATION

(11) **EP 0 585 588 B1**
(45) Date of publication and mention of the grant of the patent: **22.01.1997**
(21) Application number: 93111625.5
(22) Date of filing: 21.07.1993
(51) Int. Cl.: A47C 27/00, A61H 15/00

(54) **Rolling-massaging mattress or cushion**
Massagematratze oder Massagekissen
Matelas ou greiller de massage

(30) Priority: 30.08.1992 CN 92110186
(43) Date of publication of application: 09.03.1994
(73) Proprietor: Chan, Hoi Chau, Ting Kav, N.T. (HK)
(72) Inventor: Chan, Hoi Chau, Ting Kav, N.T. (HK)
(74) Representative: Grättinger & Partner

(56) References cited:
- EP-A- 0 137 072
- EP-A- 0 263 065
- EP-A- 0 470 767
- GB-A- 2 224 434
- GB-A- 2 234 439
- US-A- 4 169 466

## Description

The invention relates to a flexible support for positioning between a person to be supported and a resilient interior of a mattress or cushion as well as to mattresses and cushions which have a rolling-massaging function and a magnetic-therapeutic effect and to comparable personal and domestic articles, as well as parts thereof.

There are many kinds of prior art magnetic-therapeutic massaging mattresses, their construction becomes more and more exquisite and the magnetic-therapeutic function is improved continuously. They have a massaging function like being pressed with fingers, and a magnetic-therapeutic effect, for example like a "Magnetic Sleeping Mat" as described in US patent 5,035,017. With the prior art mattresses the massaging function is reached by applying a layer with nipple like projects, formed on a foamed synthetic resin board or, with the aid of an array of wooden beads and magnets adhered to a resilient interior. However, as the human body moves only slightly when people sleep or sit on such mattresses their massaging effect is relatively weak; also because the magnetic-therapeutic massaging layer of the known mattresses is not venilated enough people feel muggy when sleeping or sitting on them. Therefore the above mentioned prior art mattresses are not ideal, especially when used in a humid climate.

With another known rolling-massaging mattress (EP-A-0263065) between an upper covering and a resilient interior there is provided a therapeutic massaging layer including a whole array of spheres kept separately and omnidirectionally rotatably within holes through an elastic layer the thickness of which corresponds to the diameter of said spheres. Although with this known mattress ventilation is improved, its massaging effect is still very weak.

The object of the present invention is to compensate the drawbacks in the above mentioned prior art techniques and to provide a kind of rolling-massaging magnetic-therapeutic mattress or cushion having a strong massaging effect like being pressed by fingers, and not make people feel muggy when they sleep or sit on it.

The object of the present invention can be attained with a rolling-massaging magnetic-therapeutic mattress or cushion including a flexible support for positioning between a person to be supported and a resilient interior, said support comprising an array of spheres which are kept separately and and omnidirectionally rotatably in cavities of a plurality of independently fixed element frames being arranged in proper order on a locating substrate. Said spheres part of which are magnetic beads are separately contained in some cavities of element frames, rolling omni-directionally. The element frames are arranged in proper order on the locating substrate and fixed thereto, forming thereon an evenly embossed, well-balanced surface of rolling bodies. Their rolling action creates soothing massaging movement accompanied by strengthening blood circulation.

In the accompanying drawings some preferred embodiments are illustrated as follows:
- Fig. 1: is a diagrammatic construction sketch of the rolling-massaging mattress according to the present invention;
- Fig. 2: is a decomposed diagrammatic sketch of the element frame in the magnetic-therapeutic massaging layer of such mattress;
- Fig. 3: is a decomposed projection drawing and an assembled one of the element frame;
- Fig. 4: is a vertical view of the magnetic-therapeutic massaging layer of the mattress, of which the upper covering is taken away;
- Fig. 5: is a diagrammatic sketch of the cushion and the back of a chair according to an embodiment of the invention;
- Fig. 6: is a diagrammatic construction sketch of a cushion of a chair according to the invention, of which the upper covering is partially taken away;
- Fig. 7: is a diagrammatic construction sketch of a pillow according to the invention, of which the upper covering is taken away.

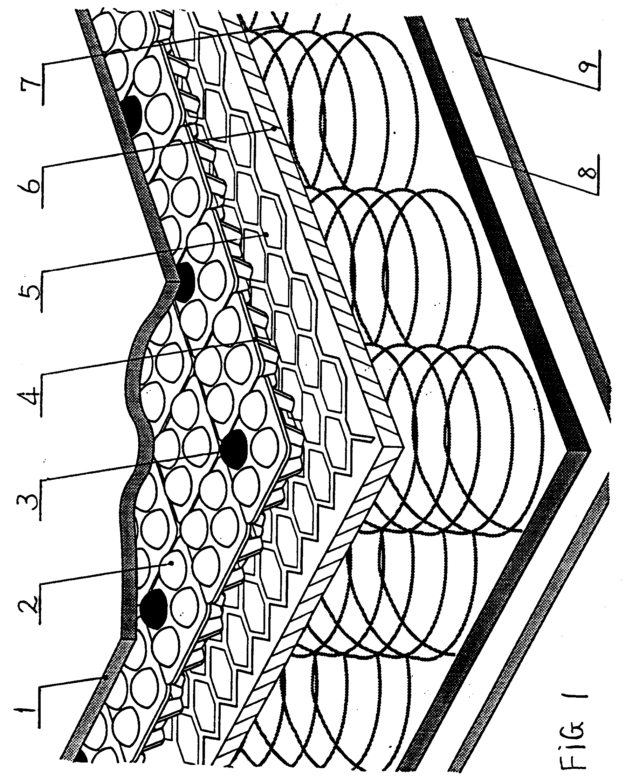

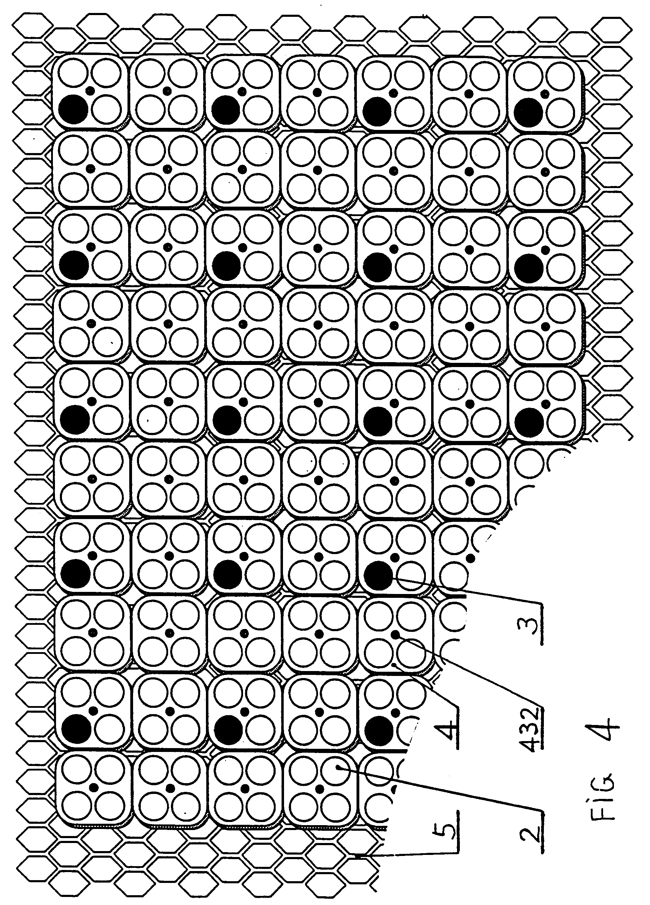

With reference to Fig. 1 of the accompanying drawings, the mattress according to the invention comprises an upper covering 1, an intermediate layer 6, a resilient interor 7, a compound liner 8 and a bottom covering 9. Between the upper covering 1 and the intermediate layer 6 there is provided a magnetic-therapeutic massaging layer including balls 2, magnetic beads 3, element frames 4 and a locating substrate 5. The balls 2 and magnetic beads 3 are contained in some cavities of element frames 4, rolling omnidirectionally. Many element frames 4 are arranged in proper order on the locating substrate 5 and fixed thereto, forming thereon a whole array of rolling bodies as shown in Fig. 4. The resilient interior 7 of the mattress or chair according to a preferred embodiment of the invention is generally a spiral steel spring matrix as shown in Fig. 1, so as to ventilate.

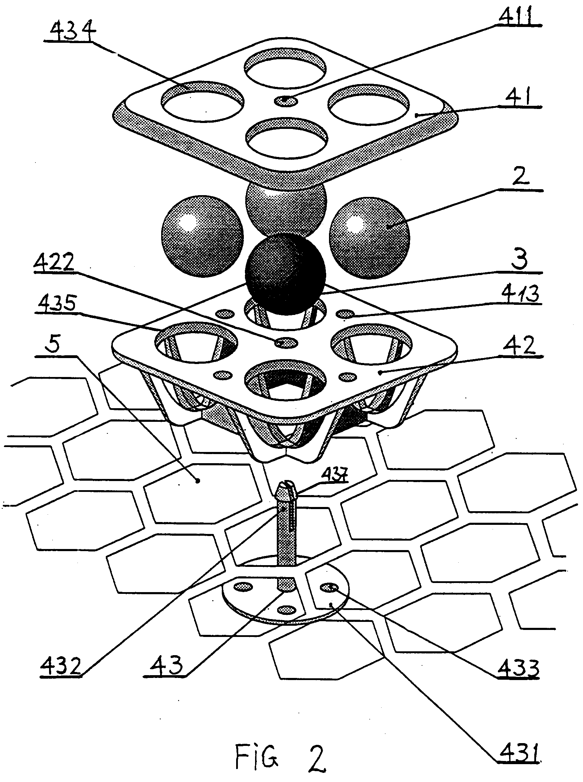

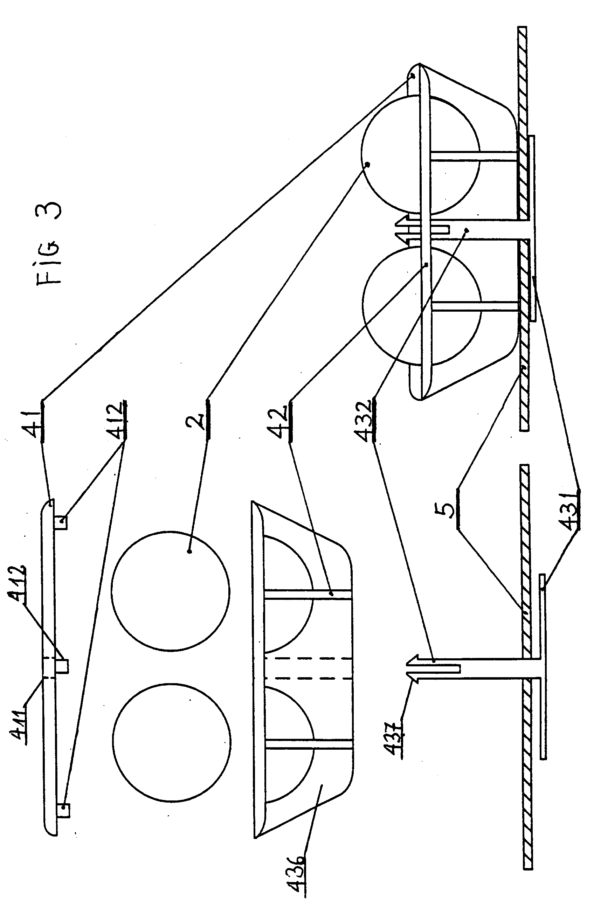

As shown in Figures 2 and 3, the element frame 4 of mattress or cushion according to the invention comprises an upper lid 41, a frame body 42 and a fixing nail 43; the balls 2, as well as the magnetic beads 3, if any, are put into each cavity of the frame body 42 separately, and then the upper lid 41 is put onto the frame body 42, the seam being welded with the aid of ultrasonic welding. Bodies of each ball 2 and magnetic bead 3 are all projecting partially from their upper lid 41, but cannot come off therefrom, and as a result forming thereby a rolling-massaging surface. Rod 432 of the fixing nail 43 passes through a mesh hole 450 from the bottom of the locating substrate 5, passes also throuh the central fixing hole 422 of the frame body 42, and protrudes from the central hole 411 of the upper lid 41, thereby latching the element frame 4 onto the locating substrate 5 with the aid of a split inverted hook 437 at the top of the nail rod 432.

The upper lid 41 of the element frame 4 is roughly a rectangular sheet, on which there are, for example, four holes 434 in order to project the rolling surfaces of the balls 2 and magnetic bead 3 and not to get them coming off; there is at the centre of the upper lid 41 a hole 411; at the underneath surface of the upper lid 41 a few of pins 412 are disposed for locating purposes. The upper part of the frame body 42 of the said element frame 4 is similar in form to the upper lid 41, only its holes 435 being larger so as to receive the balls 2 and the magnetic bead 3 nicely. There is in the centre of the upper part of the frame body 42 a hole 422 so that the rod 432 of the fixing nail 43 may pass through it. There are around said hole 422 several caves 413 into which the pins 412 on the bottom surface of the upper lid 41 can be inserted for locating. In the lower part of the frame body 42, for example, four cavities are provided to be formed of some grille-like ribs 436 for receiving the balls 2 or the magnetic bead 3. As to the fixing nail 43 of the element frame 4, its base is a sheet-like flat head 431 which can be round, with several ventilating holes 433 in it. In the centre of the flat head 431 there is disposed said nail rod 432, the top of which is an inverted hook 437 which is split in the middle.

All the components made up into the element frame 4, such as the upper lid 41, the frame body 42 and the fixing nail 43 can be formed with the aid of an injection mold from a kind of high-strength engineering plastics, for example, ABS. The ball 2 contained in the cavity of the element frame 4 is a solid or porous spheroid, and can be wooden, ceramic, of bamboo, solid- or aerated-plastic, or sintered with stone. The magnetic bead 3 contained in the said cavity is a kind of permanently magnetic spheroid, which can be made of, for example, ferrite or alnico (Al, Ni, Cu) having a surface field-intensity of from 500 to 2,000 Gauss.

In the rolling bodies array of the magnetic-therapeutic layer in a mattress according to the invention the number proportion of the balls 2 to the magnetic beads 3 is several to one, up to tens to one. The optimum is ten and several to one, for example, there are disposed in the said array only one row or one column of the miscellaneous line consisting of the balls 2 and magnetic beads 3 every three lines as shown in Fig. 4.

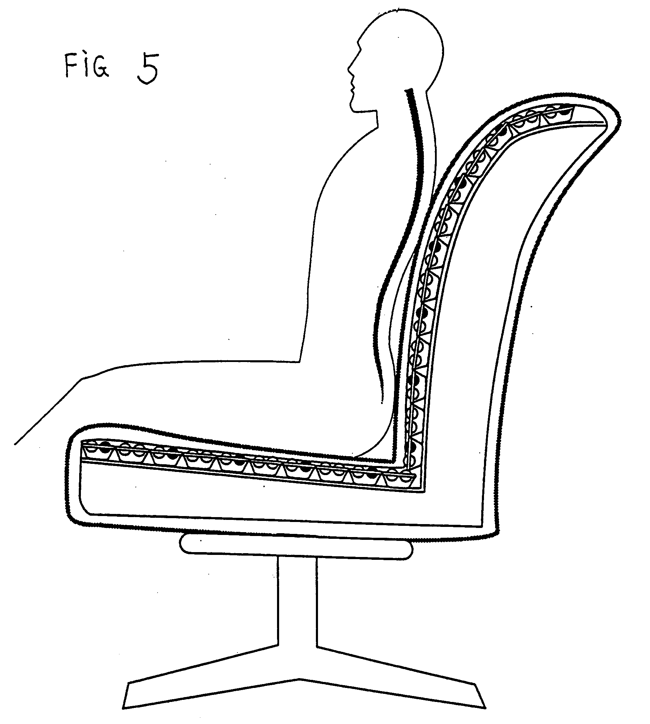

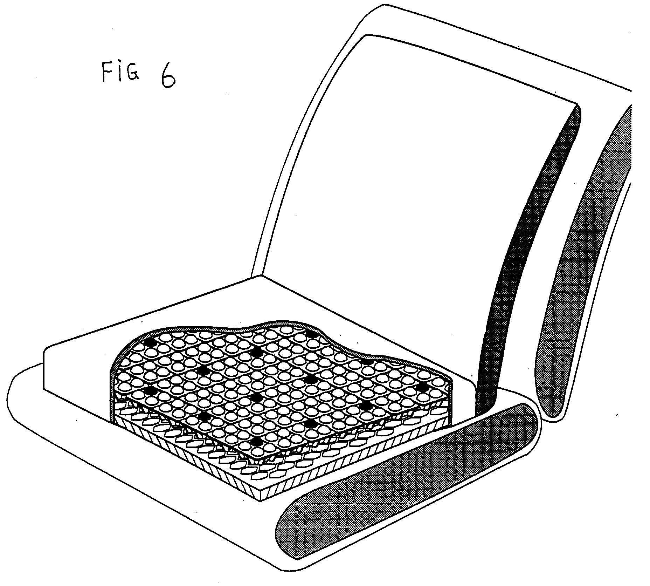

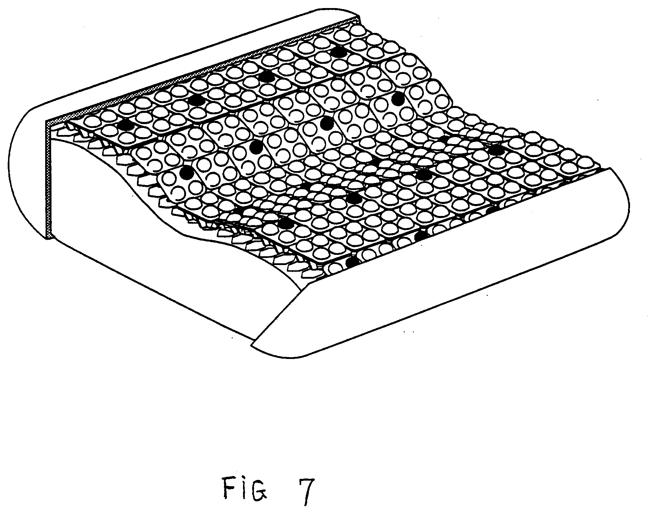

The cushion and the back according to an embodiment of the invention, is shown in Figures 5 and 6, and the pillow according to an embodiment of the invention is shown in Fig. 7. The pillow has a waved cross section, to let people feel extremely comfortable. As to both embodiments the said resilient interior 7 is often made of a plastics foam material or cocoanut fibre etc.

Using a mattress or cushion according to the invention will not only achieve the well-known magnetic-therapeutic result, but has also a strong finger-pressing massaging effect.

In the magnetic-therapeutic layer of a mattress or cushion according to the invention every rolling sphere can roll omnidirectionally. There is a frame body 42 of element frames 4 underneath the rolling spheres 2, 3, whereby several cavities are made up of some grille-like ribs and contain a lot of air. The upper covering 1 on the rolling spheres 2, 3 being rather thin the human skin strongly feels the rolling massaging effect although the human body is moving only slightly; besides, the massaging effect makes air circulating, thus reducing the temperature of human skin. As the rolling massage produces less electrostatic charge, the mattress or cushion is very comfortable. When the spiral steel spring matrix is used for the resilient interior 7, the ventilating effect is further enhanced by avoiding drawbacks from escaping chemicals in the form of decomposed products from the resilient interior 7 consisting of plastics foam material. Measurements have shown that the temperature of the contacting areas between the human skin and the mattress or cushion is up to 40°C when people sleep or sit on a common mattress or cushion, but only 35°C when people sleep or sit on a mattress or cushion according to the invention, which therefore will be more comfortable in a room without air-conditioner, especially in summer.

Compared with the prior art, the advantages of the rolling massaging mattress or cushion according to the present invention are as follows: people can not only achieve the magnetic-therapeutic result, but especially experience a strong finger-pressing massaging effect. The said mattress or cushion has good ventilation, produces less electrostatic charge, is avoiding escape of chemicals, and is especially suitable for office workers and people suffering from skin diseases.

## Claims

1. A flexible support for positioning between a person to be supported and a resilient interior (7) of a mattress or cushion comprising an array of spheres which are kept separately and omnidirectionally rotatably in cavities of a plurality of independently fixed element frames (4) being arranged in proper order on a locating substrate.

2. A rolling-massaging mattress or cushion comprising at least an upper covering (1), a resilient interior (7), a bottom covering (9) and between the upper covering (1) and the resilient interior a therapeutic massaging layer including a whole array of spheres kept separately and omnidirectionally rotatably on a locating substrate (5),
characterized in that said spheres are kept in cavities of element frames (4) being arranged separately and fixed in proper order on the locating substrate (5) and that part of said spheres consist of magnetic material.

3. Mattress or cushion according to claim 2, characterized in that each element frame (4) comprising an upper lid (41) and a frame body (42) which are connected by fixing means, each sphere projecting partially from said upper lid (41) while being rotatably kept within said element frame (4).

4. Mattress or cushion according to claims 2 and 3, characterized in that a number of cavities are formed in each frame body (42).

5. Mattress or cushion according to claim 3, characterized in that fixing means comprising a fixing nail (43) passing through said upper lid (41) and said frame body (42).

6. Mattress or cushion according to claim 5, characterized in that said fixing nail (43) being fixed in said locating substrate (5) and passing through a central fixing hole (422) of the frame body (42) and a central bore (411) of the upper lid (41).

7. Mattress or cushion according to claim 6, characterized in that said fixing nail (43) comprising a rod (432) and a split inverted hook (437) at the top of it, which fixingly projects from the central bore (411) of the upper lid (41).

8. Mattress or cushion according to claim 6, characterized in that on its lower end said fixing nail (43) having a flat head (431) being positioned underneath the locating substrate (5) thereby said rod (432) passing through a mesh hole (450) of said substrate (5).

9. Mattress or cushion according to claim 8, characterized in that several ventilating holes (433) are provided within said flat head (431).

10. Mattress or cushion according to claim 3, characterized in that said upper lid (41) which forms the upper part of said element frame (4) is roughly a rectangular sheet having symetrically positioned holes (434) to allow said spheres to partly project through forming the rolling massaging surface.

11. Mattress or cushion according to claim 10, characterized in that at the lower surface of said upper lid (41) a few pins (412) are positioned for locating said upper lid (41) with respect to corresponding caves (413) in said frame body (42).

12. Mattress or cushion according to claim 3, characterized in that said frame body (42) which forms the lower part of said element frame (4) is similar in form to the upper lid (41) having holes (435) to receive said spheres which are positioned in cavities made up of grille-like ribs (436).

13. Mattress or cushion according to claim 2, characterized in that four spheres are positioned in each element frame (4) one of which may consist of magnetic material.

14. Mattress or cushion according to claim 3, characterized in that after positioning of the spheres into element frame (4) its upper lid (41) and its frame body (42) are welded together at their common seam.

15. Mattress or cushion according to anyone of claims 2 to 14, characterized in that all the components made up into the element frame (4) such as the upper lid (41), the frame body (42) and the fixing nail (43) are formed by injection molding from a kind of high-strength engineering plastics.

16. Mattress or cushion according to claim 2, characterized in that said non magnetic spheres are balls (2) in form of a solid or porous body, which can be wooden or made of ceramic-, bamboo- or plastic material or of sintered stone.

17. Mattress or cushion according to claim 2, characterized in that the said magnetic spheres are a kind of permanently magnetic bead (3), made of ferrite or alnico (Al, Ni, Cu) and having a surface field-intensity of from 500 to 2,000 Gauss.

18. Mattress or cushion according to anyone of claims 2 to 17, characterized in that of the spheres array of the said magnetic-therapeutic massaging layer the number proportion of the said balls (2) to said magnetic beads (3) is several to one, up to tens to one.

19. Mattress or cushion according to claim 2, characterized in that the said resilient interior (7) of the mattress or chair is essentially consisting of a spiral steel spring matrix.

20. A rolling-massaging mattress or cushion comprising from upside to downside an upper covering (1), a magnetic-therapeutic massaging layer, an intermediate layer (6), a resilient interior (7), a compound liner (8) and a bottom covering (9); said magnetic-therapeutic massaging layer including balls (2), magnetic beads (3), element frames (4) and a locating substrate (5); the balls (2) and magnetic beads (3) being contained in some cavities of element frames (4), rolling omnidirectionally; the element frames (4) being arranged separately in proper order on the locating substrate (5) and fixed there, and forming therein a whole array of rolling bodies.

## Patentansprüche

1. Eine flexible Stütze zum Positionieren zwischen einer zu stützenden Person und einem federnden Innenkern (7) einer Matratze oder eines Kissens, die eine Anordnung von Kugeln umfaßt, die voneinander getrennt und in allen Richtungen drehbar in Vertiefungen einer Vielzahl von unabhängig voneinander befestigten Elementenrahmen (4) gehalten werden, die in ordentlicher Anordnung auf einem haltenden Trägergewebe angeordnet sind.

2. Massagematratze oder Massagekissen, umfassend wenigstens eine obere Abdeckung (1), einen federndern Innenkern (7), eine untere Abdeckung (9) und zwischen der oberen Abdeckung (1) und dem federnden Innenkern eine therapeutische Massageschicht, die eine ganze Anordnung von Kugeln umfaßt, die voneinander getrennt und in allen Richtungen drehbar auf einem haltenden Trägergewebe (5) gehalten werden,
dadurch gekennzeichnet,
daß die Kugeln in Vertiefungen von Elementenrahmen (4) gehalten werden, die voneinander getrennt angeordnet und in ordentlicher Anordnung auf dem haltenden Trägergewebe (5) befestigt sind, und daß ein Teil der Kugeln aus magnetischem Material besteht.

3. Matratze oder Kissen nach Anspruch 2,
dadurch gekennzeichnet,
daß jeder Elementenrahmen (4) einen oberen Deckel (41) und einen Rahmenkörper (42) umfaßt, die durch Befestigungselemente miteinander verbunden sind, wobei jede Kugel teilweise über den oberen Deckel (41) hervorsteht, während sie drehbar innerhalb des Elementenrahmens (4) gehalten wird.

4. Matratze oder Kissen nach den Ansprüchen 2 und 3,
dadurch gekennzeichnet,
daß eine Anzahl von Vertiefungen in jedem Rahmenkörper (42) gebildet ist.

5. Matratze oder Kissen nach Anspruch 3,
dadurch gekennzeichnet,
daß das Befestigungselement einen Befestigungsnagel (43) umfaßt, der durch den oberen Deckel (41) und den Rahmenkörper (42) hindurchstößt.

6. Matratze oder Kissen nach Anspruch 5,
dadurch gekennzeichnet,
daß der Befestigungsnagel (43) in dem haltenden Trägergewebe (5) befestigt ist und durch ein zentrales Befestigungsloch (422) des Rahmenkörpers (42) und eine zentrale Bohrung (411) des oberen Deckels (41) hindurchstößt.

7. Matratze oder Kissen nach Anspruch 6,
dadurch gekennzeichnet,
daß der Befestigungsnagel (43) eine Stange (432) und einen geteilten Umkehrhaken (437) an ihrer Spitze umfaßt, der befestigend von der zentralen Bohrung (411) des oberen Deckels (41) vorsteht.

8. Matratze oder Kissen nach Anspruch 6,
dadurch gekennzeichnet,
daß der Befestigungsnagel (43) an seinem unteren Ende einen flachen Kopf (431) aufweist, der unterhalb des haltenden Trägergewebes angeordnet ist, wodurch die Stange (432) durch ein Maschenloch (450) des Trägergewebes (5) hindurchstößt.

9. Matratze oder Kissen nach Anspruch 8,
dadurch gekennzeichnet,
daß mehrere Belüftungslöcher (433) in dem flachen Kopf (431) vorgesehen sind.

10. Matratze oder Kissen nach Anspruch 3,
dadurch gekennzeichnet,
daß der obere Deckel (41), der den oberen Teil des Elementenrahmens (4) bildet, im allgemeinen eine rechteckige Platte ist, die symmetrisch angeordnete Löcher (434) aufweist, durch welche die Kugeln teilweise vorstehen können, was die Massageoberfläche bildet.

11. Matratze oder Kissen nach Anspruch 10,
dadurch gekennzeichnet,
daß an der unteren Oberfläche des oberen Deckels (41) einige Stifte (412) angeordnet sind, um den oberen Deckel (41) bezüglich zugehöriger Vertiefungen (413) in dem Rahmenkörper (42) zu fixieren.

12. Matratze oder Kissen nach Anspruch 3,
dadurch gekennzeichnet,
daß der Rahmenkörper (42), der den unteren Teil des Elementenrahmens (4) bildet, in seiner Form dem oberen Deckel (41) ähnelt, der Löcher (435) aufweist, um die Kugeln, die in Vertiefungen angeordnet sind, die aus gitterartigen Rippen (436) bestehen, aufzunehmen.

13. Matratze oder Kissen nach Anspruch 2,
dadurch gekennzeichnet,
daß in jedem Elementenrahmen (4) jeweils vier Kugeln angeordnet sind, von denen eine aus magnetischem Material bestehen kann.

14. Matratze oder Kissen nach Anspruch 3,
dadurch gekennzeichnet,
daß der obere Deckel (41) und der Rahmenkörper (42) an ihrer gemeinsamen Naht zusammengeschweißt werden, nachdem die Kugeln in den Elementenrahmen (4) in ihre Lage gebracht worden sind.

15. Matratze oder Kissen nach einem der Ansprüche 2 bis 14,
dadurch gekennzeichnet,
daß alle Bestandteile, aus denen der Elementenrahmen (4) gemacht wird, wie der obere Deckel (41), der Rahmenkörper (42) und der Befestigungsnagel (43) durch Spritzgießen aus einer Art hochfestem technischem Kunststoff hergestellt werden.

16. Matratze oder Kissen nach Anspruch 2,
dadurch gekennzeichnet,
daß die nicht-magnetischen Kugeln Kugeln (2) in Form eines festen oder porösen Körpers sind, der aus Holz, Keramik, Bambus, Kunststoff oder gesintertem Stein bestehen kann.

17. Matratze oder Kissen nach Anspruch 2,
dadurch gekennzeichnet,
daß die magnetischen Kugeln eine Art von dauermagnetischen Perlen (3) aus Ferrit oder Alnico (Al, Ni, Co) sind und eine Oberflächenfeldstärke von 500 bis 2000 Gauss haben.

18. Matratze oder Kissen nach einem der Ansprüche 2 bis 17,
dadurch gekennzeichnet,
daß bei der Anordnung der Kugeln der magnetischen therapeutischen Massageschicht das Zahlenverhältnis der Kugeln (2) zu den magnetischen Perlen (3) einige zu eins bis zehn zu eins beträgt.

19. Matratze oder Kissen nach Anspruch 2,
dadurch gekennzeichnet,
daß der federnde Innenkern (7) der Matratze oder des Stuhls im wesentlichen aus einer spiralförmigen Sprungfedermatrix besteht.

20. Massagematratze oder Massagekissen, umfassend von oben nach unten eine obere Abdeckung (1), eine magnetisch-therapeutische Massageschicht, eine Zwischenschicht (6), einen federnden Innenkern (7), eine Zwischenlage (8) und eine Bodenabdeckung (9); die magnetisch- therapeutische Massageschicht umfaßt Kugeln (2), magnetische Perlen (3), Elementenrahmen (4) und ein haltendes Trägergewebe (5); die Kugeln (2) und die magnetischen Perlen (3) sind in einigen Vertiefungen von Elementenrahmen (4) enthalten und rollen in allen Richtungen; die Elementenrahmen (4) sind voneinander getrennt in ordentlicher Anordnung auf dem haltenden Trägergewebe (5) angeordnet und darauf befestigt und bilden darin eine ganze Anordnung von Rollkörpern.

## Revendications

1. Support flexible destiné à être positionné entre une personne devant être supportée et un intérieur élastique (7) d'un matelas ou d'un coussin comprenant un réseau de sphères qui sont retenues séparément et de manière à pouvoir tourner dans tous les sens, dans les cavités d'une pluralité d'éléments de cadre (4) fixés de façon indépendante et qui sont disposés selon un ordre correct sur un substrat de positionnement.

2. matelas ou coussin appliquant un massage avec roulement, comprenant au moins un revêtement supérieur (1), un intérieur élastique (7), un revêtement inférieur (9) et, entre le revêtement supérieur (1) et l'intérieur élastique, une couche de massage thérapeutique comprenant un réseau complet de sphères retenues séparément et de manière à pouvoir tourner de façon omnidirectionnelle sur un substrat de positionnement (5),
caractérisé en ce que lesdites sphères sont retenues dans des cavités d'éléments de cadre (4) disposés séparément et fixés selon un ordre correct sur le substrat de positionnement (5) et qu'une partie desdites sphères sont formées d'un matériau magnétique.

3. Matelas ou coussin selon la revendication 2, caractérisé en ce que chaque élément de cadre (4) comporte un couvercle supérieur (41) et un corps de cadre (42), qui sont raccordés par des moyens de fixation, chaque sphère étant partiellement en saillie par rapport audit couvercle supérieur (41), tout en étant retenue de manière à pouvoir tourner à l'intérieur dudit élément de cadre (4).

4. Matelas ou coussin selon les revendications 2 et 3, caractérisé en ce qu'un certain nombre de cavités sont formées dans chaque corps de cadre (42).

5. Matelas ou coussin selon la revendication 3, caractérisé en ce que les moyens de fixation comprennent une pointe de fixation (43) traversant ledit couvercle supérieur (41) et ledit corps de cadre (42).

6. Matelas ou coussin selon la revendication 5, caractérisé en ce que ladite pointe de fixation (43) est fixée dans ledit substrat de positionnement (5) et traverse un trou central de fixation (422) du corps de cadre (42) et un trou central (411) du couvercle supérieur (41).

7. Matelas ou coussin selon la revendication 6, caractérisé en ce que ladite pointe de fixation (43) comprend une tige (432) et, à la partie supérieure de cette tige, un crochet fendu retourné (437), qui fait saillie, en produisant une action de fixation, hors du trou central (411) du couvercle supérieur (41).

8. Matelas ou coussin selon la revendication 6, caractérisé en ce qu'à son extrémité inférieure, ladite pointe de fixation (43) possède une tête plate (431) qui est disposée au-dessous du substrat de positionnement (5), ladite tige (432) traversant un trou de maille (450) dudit substrat (5).

9. Matelas ou coussin selon la revendication 8, caractérisé en ce que plusieurs trous d'aération (433) sont prévus à l'intérieur de ladite tête plate (431).

10. Matelas ou coussin selon la revendication 3, caractérisé en ce que ledit couvercle supérieur (41), qui constitue la partie supérieure dudit élément de cadre (4), est approximativement une feuille rectangulaire possédant des trous (434) disposés symétriquement et permettant auxdites sphères de faire saillie en partie hors de ces trous en formant la surface de massage à roulement.

11. Matelas ou coussin selon la revendication 10, caractérisé en ce qu'au niveau de la surface supérieure dudit couvercle supérieur (41) sont disposés un petit nombre de tétons (412), qui servent à positionner ledit couvercle supérieur (41) par rapport à des cavités correspondantes (413) ménagées dans ledit corps de cadre (42).

12. Matelas ou coussin selon la revendication 3, caractérisé en ce que ledit corps de cadre (42), qui constitue la partie inférieure dudit élément de cadre (4) a une forme similaire au couvercle supérieur (41) comportant des trous (435) servant à loger lesdites sphères, qui sont positionnées dans des cavités formées par des nervures (436) disposées sous la forme d'une grille.

13. Matelas ou coussin selon la revendication 2, caractérisé en ce que quatre sphères sont positionnées dans chaque élément de cadre (4), l'une d'elles pouvant être constituée et formée d'un matériau magnétique.

14. Matelas ou coussin selon la revendication 3, caractérisé en ce qu'après le positionnement des sphères dans l'élément de cadre (4), le couvercle supérieur (41) et le corps (42) de cet élément de cadre sont réunis par soudage au niveau de leur joint de jonction commun.

15. Matelas ou coussin selon l'une quelconque des revendications 2 à 14, caractérisé en ce que tous les composants intervenant dans la constitution de l'élément de cadre (4), tels que le couvercle supérieur (41), le corps de cadre (42) et la pointe de fixation (43) sont formés par moulage par injection d'un type de matière plastique technique à haute résistance.

16. Matelas ou coussin selon la revendication 2, caractérisé en ce que lesdites sphères amagnétiques sont des boules (2) se présentant sous la forme d'un corps solide ou poreux, qui peut être en bois ou formé d'une céramique, en bambou ou en matière plastique ou de la pierre frittée.

17. Matelas ou coussin selon la revendication 2, caractérisé en ce que lesdites sphères amagnétiques sont formées par un type de bille magnétique (3) à aimantation permanente, formée de ferrite ou d'alnico (Al, Ni, Cu) ayant une intensité de champ de surface comprise entre 500 et 2000 gauss.

18. Matelas ou coussin selon l'une quelconque des revendications 2 à 17, caractérisé en ce que parmi le réseau de sphères de ladite couche magnétique de massage thérapeutique, le rapport du nombre desdites boules (2) au nombre de billes magnétiques (3) est compris entre quelques billes à une et jusqu'à dix à une.

19. Matelas ou coussin selon la revendication 2, caractérisé en ce que ledit intérieur élastique (7) du matelas ou du fauteuil est constitué essentiellement par une matrice de ressorts hélicoïdaux en acier.

20. Matelas ou coussin de massage avec roulement comprenant de haut en bas, un revêtement supérieur (1), une couche magnétique de massage thérapeutique, une couche intermédiaire (6), un intérieur élastique (7), un habillage composite (8) et un revêtement inférieur (9); ladite couche magnétique de massage thérapeutique comprenant des billes (2), des billes magnétiques (3), des éléments de cadre (4) et un substrat de positionnement (5); les boules (2) et les billes magnétiques (3) étant logées dans certaines cavités d'éléments de cadre (4), de manière à pouvoir tourner dans tous les sens; les éléments de cadre (4) étant disposés séparément et dans un ordre correct sur le substrat de positionnement (5) et étant fixés à ce dernier et formant, dans ce dernier, un réseau complet de corps roulants.
